# EUROPÄISCHE PATENTSCHRIFT

(11) **EP 2 838 871 B1**
(45) Veröffentlichungstag und Bekanntmachung des Hinweises auf die Patenterteilung: **10.08.2016**
(21) Anmeldenummer: 13715694.9
(22) Anmeldetag: 12.04.2013
(51) Int. Cl.: C07C 29/124, C07C 33/02, C07C 67/00, C07C 69/145, C07C 69/007, C07C 17/08, C07C 21/215, C07C 1/34, C07C 1/04, C07C 13/04, C07D 307/92

(54) **VERFAHREN ZUR HERSTELLUNG VON (3E, 7E)-HOMOFARNESOL**
METHOD FOR MANUFACTURING (3E, 7E) HOMOFARNESOL
PROCÉDÉ DE FABRICATION DE (3E, 7E)-HOMOFARNESOL

(30) Priorität: 16.04.2012 EP 12164279
(43) Veröffentlichungstag der Anmeldung: 25.02.2015
(73) Patentinhaber: BASF SE, 67056 Ludwigshafen am Rhein (DE)
(72) Erfinder: BONNEKESSEL, Melanie, 67067 Ludwigshafen (DE); ERNST, Hansgeorg, 76889 Schweighofen (DE); SIEGEL, Wolfgang, 67117 Limburgerhof (DE); WINTERFELDT, Ekkehard, 30916 Isernhagen (DE); HOFFMANN, Reinhard, W., 35041 Marburg (DE)
(74) Vertreter: BASF IP Association
(86) Internationale Anmeldenummer: PCT/EP2013/057653
(87) Internationale Veröffentlichungsnummer: WO 2013/156398

(56) Entgegenhaltungen:
- WO-A2-2010/139719
- JULIA M ET AL: "Préparation de composés terpéniques et apparentés, à partir de méthyl cyclopropyl cétone", BULLETIN DE LA SOCIETE CHIMIQUE DE FRANCE, SOCIETE FRANCAISE DE CHIMIE. PARIS, FRANCE, 1960, Seiten 1072-1079, XP002225028, ISSN: 0037-8968
- MIN SHI ET AL: "Lewis Acid-Catalyzed Ring-Opening Reactions of Methylenecyclopropanes with Alcoholic or Acidic Nucleophiles", ORGANIC LETTERS, Bd. 4, Nr. 13, 5. Juni 2002 (2002-06-05), Seiten 2145-2148, XP055042932, ISSN: 1523-7060, DOI: 10.1021/ol0259544
- BO XU ET AL: "Ring-Opening Reactions of Methylenecyclopropanes Promoted by Metal Halides", ORGANIC LETTERS, Bd. 5, Nr. 9, 5. April 2003 (2003-04-05), Seiten 1415-1418, XP055042931, ISSN: 1523-7060, DOI: 10.1021/ol034142k
- AMAL I. SIRIWARDANA ET AL: "Palladium-Catalyzed Addition of Nitrogen Pronucleophiles to Alkylidenecyclopropanes", THE JOURNAL OF ORGANIC CHEMISTRY, Bd. 70, Nr. 15, 24. Juni 2005 (2005-06-24) , Seiten 5932-5937, XP055042925, ISSN: 0022-3263, DOI: 10.1021/jo050700s
- STEPHANIE E. SEN ET AL: "Squalene analogs containing isopropylidene mimics as potential inhibitors of pig liver squalene epoxidase and oxidosqualene cyclase", JOURNAL OF MEDICINAL CHEMISTRY, Bd. 32, Nr. 9, September 1989 (1989-09), Seiten 2152-2158, XP055027991, ISSN: 0022-2623, DOI: 10.1021/jm00129a022
- KIRMSE W ET AL: "DESAMINIERUNGSREAKTIONEN, 461) ZERFALL VON 1-ALKENYLCYCLOPROPANDIAZONIUM-IONEN//DEAMI NATION REACTIONS, 461). - DECOMPOSITION OF 1-ALKENYLCYCLOPROPANEDIAZONIUM IONS", CHEMISCHE BERICHTE, VERLAG CHEMIE GMBH. WEINHEIM, DE, Bd. 120, Nr. 5, 1987, Seiten 839-846, XP009019811, ISSN: 0009-2940

## Beschreibung

Die vorliegende Erfindung betrifft neuartige Verfahren zur verbesserten Herstellung von Homofarnesol, insbesondere von (3E,7E)- Homofarnesol und Homofarnesolpräparaten mit erhöhtem Gehalt an (3E,7E)- Homofarnesol (auch bezeichnet als all E-Homofarnesol).

### Hintergrund der Erfindung

Ambrox ® stellt die Handelsbezeichnung für die enantiomerenreine Verbindung (-)-Ambrox (3aR,5aS,9aS,9bR)-3a,6,6,9a-tetramethyldodecahydronaphtho [2,1-b]furan), einem begehrten Riechstoff, dar. Natürlich vorkommendes (-)-Ambrox ist der olfaktorisch bedeutsamste Inhaltsstoff von Ambra, einem Verdauungsprodukt von Pottwalen.

Die verschiedenen Diastereomere von (-)-Ambrox haben einen ähnlichen Duft, unterscheiden sich aber teilweise in ihren Geruchsschwellen (G. Ohloff, W. Giersch, W. Pickenhagen, A. Furrer, B. Frei, Helv. Chim. Acta 68 (1985) 2022. G. Fráter, J. A. Bajgrowicz, P. Kraft, Tetrahedron 54 (1998) 7633). Die Geruchsschwelle des 3a-epi-(-)-Ambrox liegt um den Faktor 100 höher als die von (-)-Ambrox. Das 9b-epi-(-)-Ambrox hat dagegen bei fast gleicher Geruchsqualität wie (-)-Ambrox eine halb so hohe Geruchsschwelle. (+)-Ambrox ist achtmal schwächer als das natürliche Enantiomer. Das Racemat hat eine Geruchsschelle von 0,5 ppb und unterscheidet sich in seinen Tonalitäten kaum von dem des (-)-Ambrox®. (B. Schäfer Chemie in unserer Zeit 2011, 45, 374).

Homofarnesol ist ein wichtiges Zwischenprodukt von Syntheseverfahren zur Herstellung Ambrox ® (R. L. Snowden, Chemistry & Biodiversity 2008, 5, 958. J. and D. Leffingwell Specialty Chemical Magazine 2011, 30).

Insbesondere liefert die Zyklisierung des *all* E-Homofarnesols der Formel la 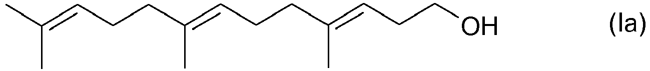 diastereomerenreines bzw enantiomerenreines Ambrox (Supersäuren: P. F. Vlad et al. Khimiya Geterotsiklicheskikh Soedinenii, Engl. Transl. 1991, 746; R. L. Snowden, Chemistry & Biodiversity 2008, 5, 958. Lewis-Säure-Brönsted-Säure: K. Ishihara et al. J. Am. Chem. Soc. 2002, 124, 3647. Mechanistische Untersuchungen: R. L. Snowden et al. J. Org. Chem. 1992, 57, 955.).

In der Literatur sind verschiedene Verfahren zur Herstellung von *all E-*Homofarnesol beschrieben:
(1) Stereoisomerenreines (3*E*,7*E*)-Homofarnesol kann aus (*E,E*)-Farnesol über (*E*, *E*)-Farnesal, C1-Verlängerung nach Wittig mit Methylentriphenyl-phosphoran und anschließender terminaler Hydroborierung des konjugierten Diens in Anlehnung an eine in der Literatur (D. S. Dodd et al. J. Org. Chem. 1992, 57, 2794) beschriebene Synthese hergestellt werden.
   Diese Synthese stellt allerdings keinen technisch-ökonomisch sinnvollen Zugang zu (*E,E*)-Homofarnesol dar. Ein technisches Verfahren zur Herstellung von isomerenreinem Farnesol ist nicht gegeben.
(2) Eine literaturbekannte Alternative zur Synthese des (3*E*,7*E*)-Homofarnesol besteht in folgendem Vorgehen (A. F. Barrero et al. J. Org. Chem. 1996, 61, 2215.): a) Destillative Trennung von (*E*/*Z*)-Nerolidol, b) Umsetzung von (E)-Nerolidol mit Dimethylformamid-dimethylacetal (DMFDMA) in einer Büchi-Umlagerung zu den korrespondierenden (3*E*/*Z*, 7*E*)-C₁₆-Amiden, c) Flash-chromatographische Trennung der stereoisomeren Amide und d) Reduktion des (3*E*,7*E*)-Amids zum korrespondierenden (3E,7E)-Homofarnesol mit Lithium-triethylborhydrid. Nachteil dieser Route sind die moderaten Ausbeuten und die erforderliche Flash-Chromatographie zur Trennung der Stereoisomeren. 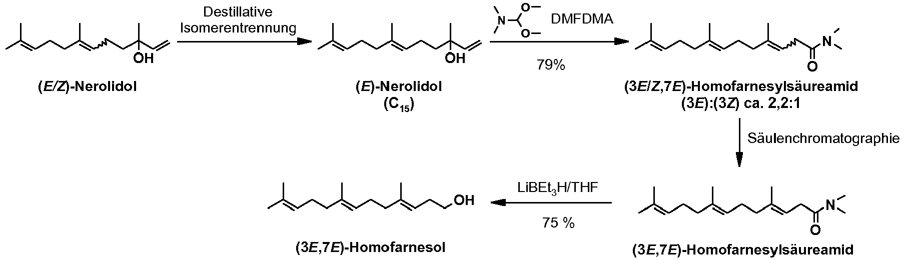
(3) In einer Patentanmeldung von Henkel (WO 92/06063, Henkel Research Corporation) wird die Carbonylierung von (*E*)-Nerolidol unter Zusatz katalytischer Mengen des relativ teuren Reagenzes Palladium(II)chlorid beschrieben. Desweiteren findet die Reaktion nachteilig für die Durchführung bei hohen CO-Drücken von ca. 70 bar statt. 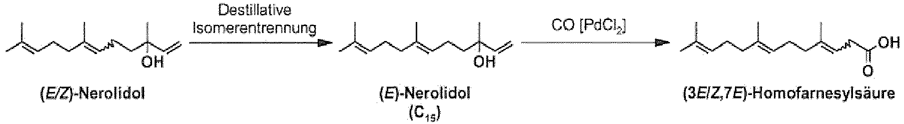
(4) Eine weitere Literaturstelle (P. Kocienski et al. J. Org. Chem. 1989, 54, 1215.) beschreibt die Synthese von Homofarnesol aus Dihydrofuran. Jeder Zyklus erfordert die Alkylierung von 5-Lithio-2,3-dihydrofuran mit einem homoallylischen Iodid gefolgt von der Ni(O)-katalysierten Kupplung mit Methylmagnesiumbromid. Das resultierende Homogeraniol kann in das korrespondierende Iodid überführt werden und der Zyklus wird wiederholt. Die Synthese ist E-selektiv und liefert das Homofarnesol über 5 Stufen in einer Gesamtausbeute von ca. 70%. 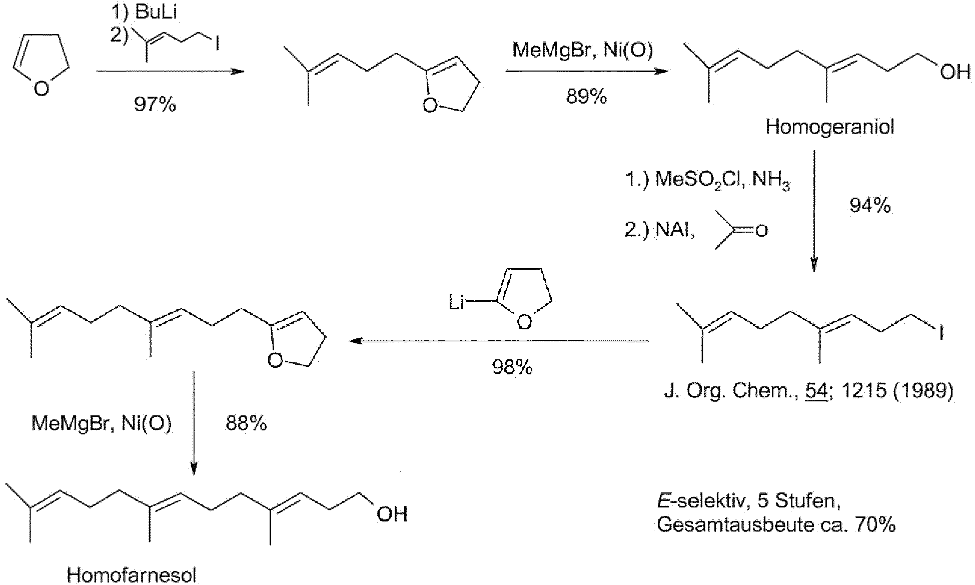
(5) Eine weitere Literaturstelle ( M. Julia, S. Julia, R. Guegan Bulletin de la Societe chimique des France, 1960, 1072-1079) beschreibt die Synthese von Homofarnesol ausgehend von Methylbromid und Acetylcyclopropan durch eine wiederholte Sequenz aus 1) Grignardbildung des Bromids, 2) Grignard-Addition an Acetylcyclopro-pan, 3) Ringöffnung des Cyclopropans und Wassereliminierung mittels HBr zum entsprechenden isoprenartigen Bromid. Die Autoren behaupten, dass die entsprechenden trans-Produkte erhalten werden, jedoch wird keine Aussage über das Produktverhältnis bei der Wassereliminierung getroffen (3 mögliche Eliminierungsrichtungen).

Mit den oben beschriebenen ersten drei Verfahren gelangt man nie direkt zum Oxidationszustand des Homofarnesols. Des Weiteren sind für die Reduktion der Homofarnesylsäure teure Hydridreagenzien erforderlich. Das Verfahren (4) ist aufgrund der erforderlichen Reagenzien wirtschaftlich nicht attraktiv.

Aufgabe der Erfindung ist daher die Bereitstellung eines verbesserten Verfahrens zur Herstellung von Homofarnesol, insbesondere (3E, 7E)-Homofarnesol, und strukturanaloger Verbindungen.

### Kurzfassung der Erfindung

Diese Aufgabe wurde allgemein durch das erfindungsgemäße Verfahren gemäß Anspruch 1 gelöst und insbesondere durch eine spezielle Ausgestaltung dieses Verfahrens zur Herstellung von (3E, 7E)-Homofarnesol.

Die Synthesestrategie beruht im Speziellen auf der Kupplung eines C₁₃- und C₃-Bausteins in einer Wittig-Reaktion. Als C₃-Baustein dient das literaturbekannte Cyclop-ropyl-phosphoniumsalz (A. Brandi et al. Chem. Rev. 1998, 589 und dort zitierte Literatur). Der C₁₃-Baustein Geranylaceton ist als Zwischenprodukt aus der Citral-Wertschöpfungskette großtechnisch und preiswert verfügbar. Das gewünschte (*E*)-Isomer ist via Destillation erhältlich. Diese Kupplungsstrategie ist auf kürzer- oder längerkettige Homologe des C₁₃-Bausteins Geranylaceton übertragbar.

### Detaillierte Beschreibung der Erfindung

### a) Allgemeine Definitionen

Werden keine gegenteiligen Angaben gemacht, so gelten folgende allgemeine Bedeutungen:
"Ambrox" umfasst insbesondere (-)-Ambrox der Formel 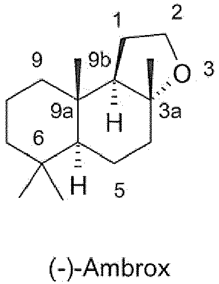 in stereoisomerenreiner Form oder gegebenenfalls im Gemisch mit wenigstens einem der folgenden Diastereomere:

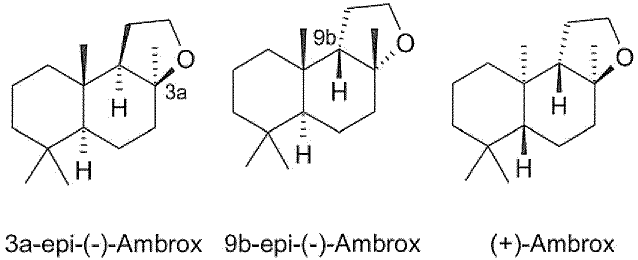

"Enantiomerenrein" bedeutet, dass neben dem speziell benannten Enantiomer keine weitere enantiomere Form einer chemischen Verbindung mit wenigstens einem Asymmetriezentrum analytisch nachweisbar ist.

"Hydrocarbyl" ist breit auszulegen und umfasst geradkettige oder ein- oder mehrfach verzweigte Kohlenwasserstoffreste mit 1 bis 50 Kohlenstoffatomen, welche ggf. zusätzlich Heteroatome, wie z.B. O, N, NH, S, in ihrer Kette enthalten können. Insbesondere steht Hydrocarbyl für geradkettige, und vor allem ein- oder mehrfach verzweigte Kohlenwasserstoffreste obiger Kettenlänge, jedoch ohne Heteroatom. Hydrocarbyl umfasst beispielsweise die im Folgenden definierten Alkyl- oder Alkenyl-Reste, insbesondere geradkettige oder verzweigte C₁-C₂₀-, C₁-C₁₀ oder C₁-C₆-Alkylreste, oder geradkettige oder verzweigte, eine- oder mehrfach, z.B. 1-, 2-, 3-, 4- oder 5-fach ungesättigte Alkenylreste mit konjugierten oder insbesondere nicht-konjugierten Doppelbindungen.

"Alkyl" steht insbesondere für gesättigte, geradkettige oder verzweigte Kohlenwasserstoffreste mit 1 bis 4, 1 bis 6, 1 bis 8, 1 bis 10, 1 bis 14 oder 1 bis 20 Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, n-Octyl, n-Nonyl und n-Decyl, n-Dodecyl, n-Tetradecyl, n-Hexadecyl, sowie die ein- oder mehrfach verzweigten Analoga davon.

"Alkoxy" steht für die OAlkyl-Analoga obiger Alkylreste,wie z.B. Methoxy, Ethoxy, n-Propoxy, 1-Methylethoxy, Butoxy, 1-Methylpropoxy, 2-Methylpropoxy oder 1,1-Dimethylethoxy; sowie z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

"Alkenyl" steht insbesondere für die ungesättigten, geradkettigen oder verzweigten Analoga obiger Alkylreste und weist insbesondere 2 bis 4, 2 bis 6, 2 bis 8, 2 bis 10, 2 bis 14 oder 2 bis 20 Kohlenstoffatome auf. Insbesondere können diese ein oder mehrfach, wie z.B. 2-, 3-, 4- oder 5-fach ungesättigt sein. Die Doppelbindungen sind dabei nicht-kumulierte Doppelbindungen. Insbesondere sind die Doppelbindungen konjugiert oder vor allem nicht-konjugiert. Beispielsweise umfasst ein geeigneter Alkenylrest gegebenenfalls repititive isoprenartige Strukturelemente 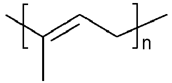 wobei n für eine ganzzahligen wert von 1 bis 8, wie z.B. 1,2,3 oder 4, stehen kann.

"Acyl" (als solches oder als Bestandteil von "OAcyl"-Resten) steht insbesondere für Reste abgeleitet von geradkettigen oder verzweigten, gegebenenfalls ein- oder mehrfach ungesättigten, gegebenenfalls substituierten C₁-C₂₄-, wie z.B. C₁-C₆- oder C₁-C₄-Monocarbonsäuren. Beispielsweise sind brauchbare Acylreste abgeleitet von folgenden Carbonsäuren: Gesättigten Säuren, wie Ameisen-, Essig-, Propion- und n- und i-Buttersäure, n- und i-Valeriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure; einfach ungesättigte Säuren, wie Acrylsäure, Crotonsäure, Palmitoleinsäure, Ölsäure und E-rucasäure; und zweifach ungesättigten Säuren, wie Sorbinsäure und Linolsäure. Sind in den Fettsäuren Doppelbindungen enthalten, so können diese sowohl in cis- als auch in trans-Form vorliegen.

"Aryl" seht insbesondere für ein- oder mehrkernige, vorzugsweise ein- oder zweikernige, insbesondere einkernige, gegebenenfalls substituierte aromatische Reste mit 6 bis 20 wie z.B. 6 bis 10 Ring-Kohlenstoffatomen, wie z.B. Phenyl, Biphenyl, Naphthyl, wie 1- oder 2-Naphthyl, Tetrahydronaphthyl, Fluorenyl, Indenyl und Phenanthrenyl. Diese Arylreste können gegebenenfalls 1, 2, 3, 4, 5 oder 6 gleiche oder verschiedene Substituenten tragen, beispielsweise ausgewählt unter Halogen, Alkyl, insbesondere mit 1 bis 4 Kohlenstoffatomen, Alkenyl, insbesondere mit 2 bis 4 Kohlenstoffatomen, OH, Alkoxy, insbesondere mit 1 bis 4 Kohlenstoffatomen, Acyl, insbesondere mit 1 bis 4 Kohlenstoffatomen, NH₂ oder NO₂.

"Halogen" steht für F, Cl, Br oder J.

### b) Spezielle Ausgestaltungen

Die vorliegende Erfindung betrifft insbesondere folgende Ausführungsformen:
1. Verfahren zur Herstellung von Verbindungen der allgemeinen Formel I 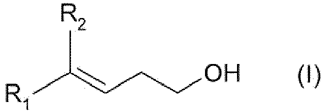 worin
   R₁ für einen geradkettigen oder verzweigten, gegebenenfalls ein- oder mehrfach (wie z.B. 1- oder 2-fach) ungesättigten, Kohlenwasserstoffrest mit 1 bis 50 Kohlenstoffatomen, z.B. C₁-C₂₀, C₁-C₁₁ oder C₁-C₆ Hydrocarbyl-, wie insbesondere einen geradkettigen oder verzweigten C₁-C₂₀-, C₁-C₁₁ oder C₁-C₆- Alkylrest oder einen geradkettigen oder verzweigten C₂-C₂₀-, C₂-C₁₀- oder C₂-C₆-Alkenylrest mit einer oder mehreren konjugierten oder insbesondere nicht-konjugierten Doppelbindung, oder z.B. für einen isoprenartigen Rest der Formel 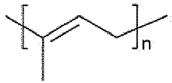 steht, worin n für 1, 2, 3, 4 oder 5 steht, wobei die Kohlenwasserstoffkette gegebenenfalls Heteroatome enthält;
   und R₂ für H oder C₁-C₆-Alkyl, insbesondere Methyl oder Ethyl steht, wobei man
   a) eine Carbonylverbindung der Formel II 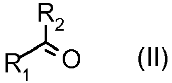 worin R₁ und R₂ die oben angegebenen Bedeutungen besitzen,
      mittels Wittig-Olefinierung zu einem Cyclopropan der allgemeinen Formel (III) 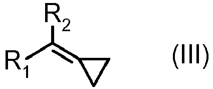 worin R₁ und R₂ die oben angegebenen Bedeutungen besitzen, umsetzt,
   b) das Cyclopropan der Formel III unter Ringöffnung zu einer Verbindung der Formel IV 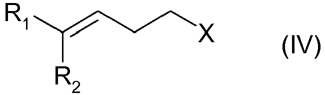 worin R₁ und R₂ die oben angegebenen Bedeutungen besitzen, und X für Halogen, wie z.B. Cl oder Br, oder O-R' steht, worin R' für H, Acyl, wie C₁-C₄-Acyl, insbesondere Acetyl, Tf-Acetyl oder SO₂-R" steht, worin R" für Alkyl, insbesondere C₁-C₄-Alkyl oder gegebenenfalls substituiertes Aryl, insbesondere gegebenenfalls substituiertes Phenyl, steht;
      umsetzt und
   c) die Verbindung der allgemeinen Formel IV in die Verbindung der allgemeinen Formel I überführt.
2. Verfahren nach Ausführungsform 1, wobei man zur Wittig-Olefinierung nach Stufe a) ein Cyclopropylphosphoniumsalz verwendet.
3. Verfahren nach Ausführungsform 2, worin das Cyclopropylphosphoniumsalz eine Triphenlyphosphonium-Verbindung der Formel V ist 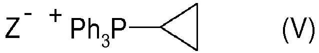 worin Z- für das Anion einer starken Säure, wie insbesondere ein Halogenid, wie z.B. Fluorid, Chlorid oder Bromid, bevorzugt Bromid, steht.
4. Verfahren nach Ausführungsform 3, wobei man die Verbindung der Formel V herstellt, indem man a) Brombutyrolacton mit Triphenylphosphin umsetzt und anschließend thermisch decarboxyliert oder b) 1,3-Dibrompropan mit Triphenylphosphin, insbesondere in Gegenwart einer Base, wie insbesondere einer Base ohne nukleophile Eigenschaften (wie z.B. PhLi, NaH, K-tert.-butylat), umsetzt und anschließend das Umsetzungsprodukt cyclisiert.
5. Verfahren nach einer der vorhergehenden Ausführungsformen, worin die Ringöffnung der Stufe b) in Gegenwart einer Lewis-Säure (wie z.B. AlCl₃, BF₃, SiCl₄, PF₅, Sn(OTf)₂, Cu(OTf)₂) bzw. Brönstedtsäure/Protonensäure (wie z.B. Ameisensäure, Essigsäure, Propionsäure, Schwefelsäure, Pivalinsäure, Isobuttersäure, Alkyl- und Arylsulfonsäuren, z.B. Methansulfonsäure oder para-Toluolsulfonsäure) und eines Nukleophils (wie z.B. OH⁻, Formiat, Acetat, Propionat, Pivalat, Isobutyrat, Alkyl- und Arylsulfonat, z.B. Methansulfonat oder para-Toluolsulfonat, Chlorid, Bromid) erfolgt, wobei Tf für Trifluormethansulfonyl steht.
6. Verfahren nach Ausführungsform 5, wobei die Ringöffnung im Wesentlichen stereoselektiv, insbesondere E-selektiv (bezüglich R₁) erfolgt. 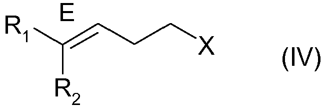 E-Selektivität ist dabei insbesondere gegeben, wenn nach Ringöffnung die E-Form in mengenmäßigen (molaren) Überschuss gebildet wird (d.h. E : Z >1, wie z.B. >1.01, wie z.B. >1.5 oder >2, wie z.B. im Bereich von 1,5 bis 100, 2 bis 50 oder 2,2 bis 10; oder E wird quantitativ, d.h. Z-Form ist analytisch nicht nachweisbar, gebildet.
7. Verfahren nach einer der vorhergehenden Ausführungsformen, wobei man in Stufe c) die Verbindung der allgemeinen Formel IV in eine Verbindung der allgemeinen Formel I überführt indem man, wenn X für OAcyl, wie z.B. OAcetyl, steht, eine Esterspaltung durchführt, oder wenn X für Halogen, wie z.B. Cl oder Br, steht, das Halogenid in eine Ester über führt, wie z.B. mit einem Salz der Ameisensäure(z.B. Natriumformiat) in den korrespondierenden Ameisensäureester überführt, und diesen Ester anschließend spaltet.
8. Verfahren nach einer der vorhergehenden Ausführungsformen, worin man ein Produkt, enthaltend ein (3*E*, 7*E*)-Homofarnesol der Formel Ia 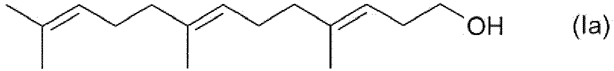 erhält.
9. Verfahren nach Ausführungsform 8, wobei man in Stufe a) E-Geranylaceton der Formel IIa 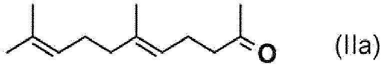 mit Cyclopropylphosphoniumhalogenid umsetzt, so dass man das Cyclopropan der Formel IIIa 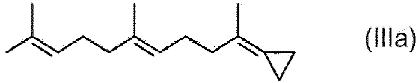 erhält.
10. Verbindung der Formel IIIa.
11. Verfahren zur Herstellung von enantiomerenreinem Ambrox oder eines Stereoisomeren-Gemischs von Ambrox, wobei man (3E, 7E)-Homofarnesol nach einem Verfahren gemäß einer der vorhergehenden Ausführungsformen, 1 bis 9 herstellt und das so gebildete Homofarnesol in an sich bekannter Weise chemisch oder enzymatisch zu enantiomerenreinem oder racemischem Ambrox oder beliebige Stereoisomeren-Mischungen hiervon umsetzt.

### c) Detaillierte Beschreibung des erfindungsgemäßen Verfahrens

Anhand einer bevorzugten Ausführungsform zur Herstellung von all E-Homofarnesol, wird das erfindungsgemäße Verfahrensprinzip, ohne auf diese spezielle Reaktion beschränkt zu sein, näher erläutert. Spezielle Ausgestaltungen sind daher auf andere eingesetzte Ausgangsverbindungen übertragbar.

Der C₁₆-Baustein (*E*)-C₁₆-Cyclopropan kann durch Wittig-Olefinierung von (*E*)-Geranylaceton mit dem Cyclopropyltriphenylphosphoniumsalz wie folgterhalten werden:

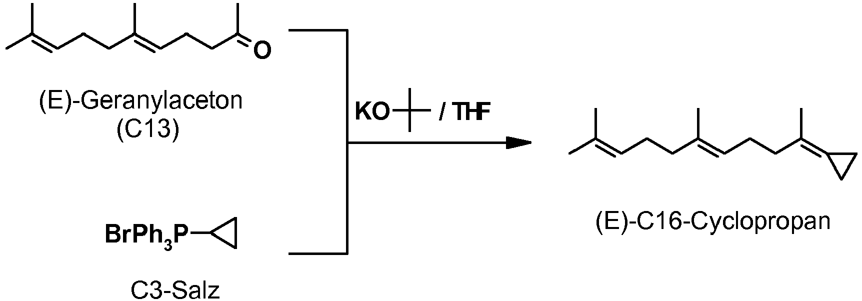

Das C₃-Salz kann in Anlehnung an Literaturvorschriften aus α-Brombutyrolacton in zwei Stufen über ein C₄-Salz als Zwischenprodukt hergestellt werden (S. Fliszár et al. Helv. Chim. Acta 1963, 46, 1580. H. J. Bestmann et al. Tetrahedron Lett. 1966, 3591. E. E. Schweizer et al. J. Chem. Soc., Chem. Comm. 1966, 666. H. J. Bestmann et al. Angewandte Chemie 1965, 77, 1011.):

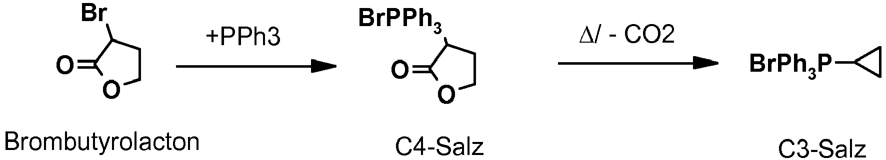

Eine Alternative zur C₃-Salzsynthese ausgehend von 1,3-Dibrompropan ist ebenfalls literaturbekannt (K. Sisido et al. Tetrahedron Lett. 1966, 3267. A. Maercker et al. Tetrahedron 1994, 50, 2439. K. Utimoto et al. Tetrahedron 1973, 29, 1169., E. E. Schweizer et al. J. Org. Chem. 1968, 33, 336.):

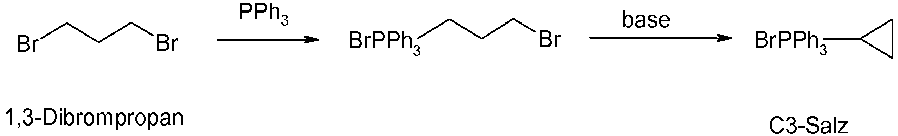

Klassische Wittig-Olefinierungen mit Cyclopropyltriphenylphosphoniumbromid sind in der Literatur nur wenig beschrieben (A. Brandi et al. Chem. Rev. 1998, 589 und dort zitierte Literatur. M. Giersig et al. Chem. Ber. 1988, 525.). Die Ausbeuten für diese Wittig-Reaktionen mit diversen Ketonen (z.B. Cyclohexanon, Benzophenon) liegen zwischen 43% und 80%. Zur Vermeidung von Nebenreaktionen werden Basen ohne nukleophile Eigenschaften (PhLi, NaH, K-tert.-butylat) eingesetzt, wie im vorliegenden Fall die relativ preiswerte Base K-tert.-butylat oder NaH. Im allgemeinen sind bei den Wittig-Reaktionen die Ausbeuten für trisubstituierte Olefine generell niedrig und bei tetrasubstituierten Olefinen nochmals schlechter (H. G. Ernst, Carotenoids, Volume 2, Synthesis, S. 80f.).

Überraschenderweise wurde erfindungsgemäß festgestellt, dass die Wittig-Olefinierung von Cyclo-propyltriphenylphosphoniumbromid mit einem aliphatischen Keton wie (E)-Geranylaceton unter Verwendung von Kalium-*tert*Butylat zum (*E*)-C₁₆-Cyclopropan in Ausbeuten > 90% verläuft (siehe Beispiel 1). Die Wittig-Reaktion kann unter Verwendung von 2 eq NaH auch ausgehend vom TPP-Brompropansalz *und in situ* Zyklisierung zum Cyclopropylphosphoniumsalz in sehr guten Ausbeute von > 90% erfolgen (siehe Beispiel 2).

Das bisher in der Literatur unbekannte (*E*)-C₁₆-Cyclopropan kann in Gegenwart einer Säure, z.B. einer Lewis-Säure wie AlCl₃ oder BF₃*Et₂O und eines Nukleophils in regio- und stereoselektiver Weise zu Homofarnesylderivaten geöffnet werden.

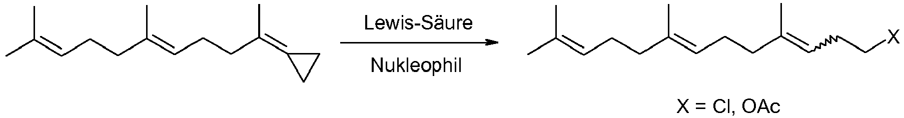

Die Ringöffnung von Alkylidencyclopropanderivaten ist in der Literatur (H. Pellissier Tetrahedron 2010, 66, 8341 und dort zitierte Literatur) beschrieben worden, allerdings wurden wie in unserem Fall geschehen keine Seitenketten mit Doppelbindungen betrachtet (siehe Beispiele 3 und 4).

Anschließend kann das Homofarnesylchlorid durch klassische Acetat-Substitution und Hydrolyse in das Homofarnesol überführt werden. Alternativ kann das Homofarnesol in Anlehnung an die Literatur (H. A. Zahalka et al. Synthesis 1986, 763.) ausgehend vom Homofarnesylchlorid über das Formiat und anschließende Hydrolyse dargestellt werden (siehe Beispiel 5).

*3E,7E- und* 3Z/7E-Homofarnesol sowie die entsprechenden 3Z-Isomere können durch Destillation getrennt werden, so dass man reines 3E,7E-Homofarnesol erhält.

Das so gebildete Homofarnesol kann dann in einem weiteren Schritt zu Ambrox cyclisiert werden (siehe Beispiel 6).

Diese Cyclisierung kann dabei in an sich bekannter Weise oder wie in den folgenden Ausführungsbeispielen beschrieben erfolgen. Sowohl enzymatische als auch chemische Zyklisierungen kommen dafür in Betracht.

So ist beispielsweise die enzymatische Cyclisierung mittels Squalen-Hopen-Cyclase aus der Wo 2010/139719 bekannt, worauf hiermit ausdrücklich Bezug genommen wird.

Chemische Cyclisierungsreaktionen unter Verwendung einer Supersäure (Fluorsulfonsäure in 2-Nitropropan) sind z.B. aus P. F. Vlad et al. Khimiya Geterotsiklicheskikh Soedinenii, Engl. Transl. 1991, 746 bekannt. Weitere Verfahren umfassen eine Cyclisierung umfassend die enantioselektive Polyencyclisierung von Homofarnesyltriethylsilylether in Gegenwart von O-(o-Fluorbenzyl)-binol und SnCl₄, wie von Yamamoto beschrieben (H. Yamamoto et al. J. Am. Chem. Soc. 2002, 3647.)

### Experimenteller Teil

### A) Material und Methoden

### HPLC-Analytik:

### Methode 1)

### Geräteeinstellungen und Chromatographische Bedingungen:

| | |
|---|---|
| Gerät : | Agilent Series 1100 |
| Säule : | Zorbax Eclipse XDB-C18 1,8µm 50*4,6mm kombiniert mit einer Zorbax |
| Extend C18 | 1,8µm 50*4,6mm von Agilent® |
| Eluent : | -A: Wasser mit 0,1Vol% H₃PO₄ |
| | -B: Acetonitril mit 0,1Vol% H₃PO₄ |

| Zeit in min | %B | Fluss |
|---|---|---|
| 0,0 | 70 | 1 |
| 10,0 | 80 | 1 |
| 13,0 | 100 | 1 |
| 17,0 | 100 | 1 |
| 17,1 | 70 | 1 |

| | |
|---|---|
| Detektor : | UV-Detektor λ=197 nm, BW=4 nm |
| Flussrate : | 1 ml/min |
| Injektion : | 1 µL |
| Temperatur : | 50°C |
| Laufzeit : | 20 min |
| Druck : | ca. 160 bar |

### Methode 2)

### Geräteeinstellungen und Chromatographische Bedingungen:

| | |
|---|---|
| Gerät : | Agilent Series 1100 |
| Säule : | Chiralpak AD-RH 5µm 150*4,6mm von Daicel® |
| Eluent : | -A: Wasser mit 0,1Vol% H₃PO₄ |
| | -B: Acetonitril mit 0,1Vol% H₃PO₄ |

| Zeit in min | %B | Fluss |
|---|---|---|
| 0,0 | 30 | 1,2 |
| 25,0 | 70 | 1,2 |
| 30,0 | 100 | 1,2 |
| 40,0 | 100 | 1,2 |
| 40,1 | 30 | 1,2 |

| | |
|---|---|
| Detektor : | UV-Detektor λ=205 nm, BW=5 nm |
| Flussrate : | 1,2 ml/min |
| Injektion : | 5 µL |
| Temperatur: | 40°C |
| Laufzeit : | 45 min |
| Druck : | ca. 70 bar |

### B) Herstellungbeispiele

### Beispiel 1: Wittigreaktion ausgehend vom Cyclopropylphosphoniumsalz mit Kalium-tertButylat

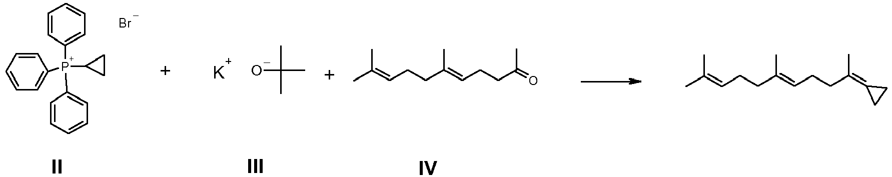

**Einsatzstoffe:**

| | | | | |
|---|---|---|---|---|
| 640ml | (7890,58mmol | **I** | Tetrahydrofuran | **24,7eq** |
| 598,96g | ) | | M = 72,11 g/mol | |
| 122,6g | (320mmol) | **II** | Cyclopropyl-Phosphoniumsalz | **1 eq** |
| | | | M = 383.27 g/mol | |
| 35,91 g | (320mmol) | **III** | Kalium-tert-butylat | **1 eq** |
| | | | M = 112,21 g/mol | |
| 48,6g | (288mmol) | **IV** | E-Geranylaceton | **0,9** |
| | | | M = 194,32 g/mol | **eq** |

Der Reaktor wird mit Stickstoff gespült. 500 mL THF **(I)** werden vorgelegt und auf 0°C abgekühlt. In der Reibschale zerkleinertes Cyclopropyl-Phosphoniumsalz **(II)** wird zugegeben, mit der Restmenge (140 ml) THF **(I)** nachgespült und bei 0°C 8 min gerührt. Unter N₂-Atmosphäre wird Kalium-tert-butylat **(III)** zugegeben, dabei steigt die Innentemperatur auf 5°C. Die Suspension wird sofort rot-orange, rührt anschließend bei ca. 0°C 2 Stunden nach.

Geranylaceton **(IV)** wird innerhalb ca. 10 min zugetropft (leichte Exothermie!), danach 15 min mit -2°C Öltemperatur nachgerührt. Anschließend wird das Reaktionsgemisch wird mit Delta 1°C (Öltemperatur zu Innentemperatur) erwärmt. Wenn eine Innentemperatur von 35°C erreicht ist, wird mit 35°C Öltemperatur über Nacht weitergerührt. Nach insgesamt 24 h Rührzeit (Umsatzkontrolle via DC: n-Heptan/EE = 10:1) wird der Ansatz aufgearbeitet. Zur Reaktionssuspension werden bei 35°C Innentemperatur 1000 ml n-Heptan zugegeben und durch Regelung des Vakuums so Destillat abgezogen, dass die Innentemperatur 35°C nicht übersteigt, beheizt wird mit Delta 15°C zur Innentemperatur. Bei einem Druck von 155 mbar wird die Öltemperatur auf 35°C abgesenkt, anschließend mit N₂ belüftet. Destillat und Kühlfalle werden entleert. (Fraktion 1: 494g, darin 259g THF nach GC A%). Die Apparatur wird auf 150 mbar evakuiert. Durch Regelung des Vakuums wird so Destillat abgezogen, dass die Innentemperatur 35°C nicht übersteigt, beheizt wird mit Delta 15°C zur Innentemperatur. Bei einem Druck von 95 mbar wird die Öltemperatur auf 20°C abgesenkt, anschließend mit N₂ belüftet. Destillat und Kühlfalle werden entleert. (Fraktion 2: 292g, darin 63g THF nach GC A%). Insgesamt wurden ca. 320g THF (nach GC A%, n-Heptan wird allerdings überbewertet) von 569 g abdestilliert.

Zum Reaktorinhalt (Suspension, 20°C) werden 530 g Wasser eingerührt, Phasentrennung nach ca. 5 min. Die Unterphase-1 wird (UP1, 395 g, hellbraun) verworfen. Die Oberphase-1 mit TPPO-Mulm wird mit 500 ml Wasser 5 min gerührt, Phasentrennung nach 5 min. Die Unterphase-2 wird (UP2, 559 g, hellbraun) verworfen. Die Oberphase-2 mit TPPO-Mulm wird mit 500 ml Wasser/Methanol (1:1 Vol-Teile, 455 g) 5 min. gerührt, der Rührer wird ausgeschaltet. Das TPPO ist nicht vollständig im Wasser/MeOH gelöst. Die Phasentrennung erfolgt nach 5 min. Unterphase-3 (UP3, 545 g, trübe Phase). Die Oberphase-3 mit TPPO-Mulm wird mit 500 ml Wasser/Methanol (1:1 Vol-Teile, 455 g) 5 min. gerührt, der Rührer wird ausgeschaltet. Das TPPO ist nun vollständig gelöst, klare Phasen! Phasentrennung erfolgt nach 5 min. Unterphase-4 (UP4, 557 g, trübe Phase). Die Oberphase-4 wird nochmals mit 500 ml Wasser/Methanol (1:1 Vol-Teile, 455 g) 5 min. gerührt, der Rührer wird ausgeschaltet. Nahezu klare Phasen sind zu beobachten! Phasentrennung erfolgt nach 5 min. Unterphase-5 (UP5, 454 g, schwach trübe Phase). Die Oberphase-5 wird nochmals mit 500 ml Wasser gewaschen, die Phasen werden getrennt. Unterphase-6 wird (UP6, 509 g, klare Phase) verworfen. Die Oberphase-6 (303 g) wird am Rotationsverdampfer einrotiert (45°C Bad, volles Ölpumpenvakuum).

Man erhält 60,4 g Wertprodukt (gelbes Öl). Das gewünschte Produkt konnte mit ca. 97,2 A% Reinheit (E&Z) und etwa 93% Ausbeute bezogen auf Geranylaceton isoliert werden, bezogen auf C3-Phosphoniumsalz beträgt die Ausbeute 84,0%.

**HPLC Methode 2**

| **RT** | **Substanz** | **FI**% |
|---|---|---|
| 6,14 | TPPO | 1,51 |
| 10,79 | E-Geranylaceton | 0,24 |
| 20,64 | Z-C16-Cyclopropan | 6,03 |
| 22,25 | E-C16-Cyclopropan | 91,21 |

### Beispiel 2: Wittigreaktion ausgehend vom TPP-Brompropansalz mit NaH

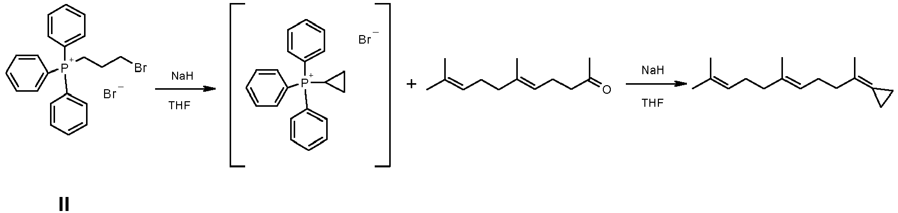

**Einsatzstoffe:**

| | | | | |
|---|---|---|---|---|
| 250ml | (3082,3mmol) | **I** | Tetrahydrofuran | **12,3eq** |
| 222,25g | | | M = 72,11 g/mol | |
| 126,6g | (250mmol) | **II** | C₄-Salz HPLC - 91,67Gew% | **1 eq** |
| 22g | (550mmol) | **III** | Natriumhydrid in Mineralöl 60%ig | **2,2eq** |
| | | | M = 24 g/mol | |
| 48,6g | 240,1 mmol | **IV** | Geranylaceton | **0,96eq** |
| | bez. auf (E) | | 90%(E) und 6%(Z) | |
| | und (Z) | | M = 194,32 g/mol | **0,9** |
| | **225 mmol** | | | **eq** |
| | **bez. auf (E)** | | | |

Das C₄-Salz **(II)** wird bei Raumtemperatur in THF(I) vorgelegt. Das Natriumhydrid **(III)** wird 3x mit jeweils 100ml n-Hexan gewaschen, im Stickstoffstrom getrocknet und zugegeben. Die weiße Suspension rührt 4,5h bei Raum-temperatur nach (Umsatzkontrolle via HPLC). Dann wird Geranylaceton **(IV)** zugegeben und für 21 h auf 35°C erhitzt. Danach wird der gelblichen Suspension 500ml n-Heptan zugesetzt. Anschließend wird am Rotationsverdampfer THF entfernt. Die verbleibende Suspension wird mit 500ml Wasser/ Methanol versetzt und die Phasen werden getrennt. Die wässrige Phase wird 2x mit 250ml n-Heptan extrahiert. Die vereinigten organischen Phasen werden 6x mit 250ml Wasser/Methanol gewaschen um gebildetes NaBr und TPPO abzutrennen. Die organische Phase wird über Natriumsulfat getrocknet und am Rotationsverdampfer bei 50°C/10mbar eingeengt. Man erhält 50,6 g Wertprodukt als braunes, klares Öl.

| Geranylaceton | 100% Ausbeute | Ausbeute (%) bez. auf Σ E&Z Gew% | | Ausbeute (%) bez. auf Σ E&Z FI% | | Ausbeute (%) bez. auf E-C₁₆-Cyclopropan FI% | |
|---|---|---|---|---|---|---|---|
| [mmol] | [g] | [g] | [%] | [g] | [%] | [g] | [%] |
| 240 | 52,4 | 49,3 | 94,2 | 49,2 | 93,9 | | |
| **225** | **49,1** | | | | | **46,1** | **94,0** |

**HPLC Methode 2:**

| **Probe :** | **36257/** | **A** |
|---|---|---|
| **RT** | **Substanz** | **FI**% |
| 2,68 | C₃-Salz | |
| 3,27 | C₄-Salz | |
| 6,05 | TPPO | |
| 10,65 | E-Geranylaceton | 0,4 |
| 20,57 | Z-C₁₆-Cyclopropan | 6,0 |
| 22,16 | E-C₁₆-Cyclopropan | 91,2 |

**HPLC Methode 1:**

| **Probe :** | **36257/** | **A** | |
|---|---|---|---|
| **RT** | **Substanz** | **FI%** | **Gew**% |
| 1,05 | C₃-Salz | | |
| 1,10 | C₄-Salz | | |
| 1,27 | TPPO | | |
| 3,64 | Geranylaceton | | |
| 14,53 | C₁₆-Cyclopropan | 97,2 | 97,5 |

### Beispiel 3: Ringöffnung mit BF₃-Etherat und Eisessig

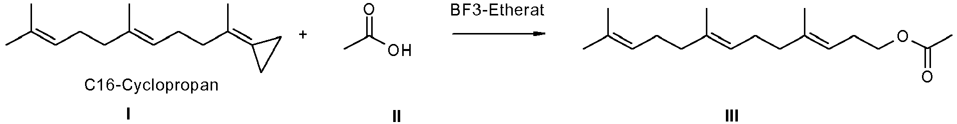

**Einsatzstoffe:**

| **Substanz** | **Reinheit %** | **Molgew. g/mol** | **Masse g** | **Stoffmenge mol** | **Eq** |
|---|---|---|---|---|---|
| C₁₆-Cyclopropan | 90,3 (bez. auf E- & Z) | 218,38 | 109,2 | 0,452 | 1 |
| Eisessig | 100 | 60,05 | 1914 | 31,875 | 70,5 |
| BF₃-Etherat | 100 | 141,93 | 9,05 | 0,0638 | 0,14 |
| Eisessig | 100 | 60,05 | 204 | 3,4 | 7,5 |

Das C₁₆-Cyclopropan (I) wird in Eisessig (II) gelöst und ergibt eine klare, gelbe Lösung. Das BF₃-Etherat wird in Eisessig vorverdünnt und innerhalb von 2min bei RT zugegeben. Die Lösung wird langsam dunkler, keine Wärmetönung. Über Nacht wird bei RT gerührt. Man erhält eine dunkelbraune, klare Lösung (HPLC-Analytik). Nach 27h bei RT werden der klaren, dunkelbraunen Lösung 2,5L Wasser und 1 L Cyclohexan zugesetzt und die Phasen getrennt. Die wässrige Phase wird zweimal mit 0,5L Cyclohexan extrahiert. Die vereinigten organischen Phasen werden nacheinander mit 4x 0,2L Wasser, 0,25L gesättigter NaHCO₃-Lösung und erneut 0,25L Wasser gewaschen. Die organische Oberphase wird über Natriumsulfat getrocknet und am Rotationsverdampfer bei 40°C/4mbar eingeengt. Man erhält 122,5g Wertprodukt als oranges, flüssiges Rohprodukt (Theorie 118,3g) (HPLC-Analytik Methode 2: A) Das Homofarnesylacetat wird in einem 3Z,7E : 3E,7E- Verhältnis von 1 : 2,45 erhalten.

Bei der ersten wässrigen Phase trennten sich über Nacht noch ca. 150mL organische Phase nach. Diese wurde isoliert und ebenfalls mehrfach mit Wasser und gesättigter NaHCO₃-Lösung gewaschen. Die organische Oberphase wird über Natriumsulfat getrocknet und am Rotationsverdampfer bei 40°C/4mbar eingeengt. Man erhält weitere 7,0 g Wertprodukt (HPLC-Analytik Methode 2: B).

| **HPLC Methode 2:** Flächen% | | | | | | | | | | |
|---|---|---|---|---|---|---|---|---|---|---|
| min | 10,70 | 14,10 | 15,18 | 16,61 | 17,78 | 18,84 | 20,13 | 20,68 | 22,27 | >28 |
| | Geranylaceton | 12-Acetoxy-Hofa-acetat Stereoisomere | | 3E/Z,7Z-Hofa-acetat | | 3Z,7E-Hofa-acetat | 3E,7E-Hofa-acetat | Z-C16-Cyclopropan | E-C16-Cyclopropan | |
| Edukt | 5,1 | 0 | 0 | 0 | 0 | 0 | 0 | 5,7 | 84,6 | 0,1 |
| A | 3,6 | 3,4 | 7,1 | 2,8 | 6,8 | 17,2 | 42,1 | 0 | 0,2 | 7,9 |
| B | 3,0 | 2,6 | 6,6 | 2,1 | 6,9 | 16,1 | 40,5 | 0 | 0,3 | 8,3 |

Das gewünschte Produkt konnte mit ca. 60% Reinheit und etwa 62% Ausbeute isoliert werden. Eine Nachextraktion der ersten wässrigen Phase konnte die Ausbeute auf 65% steigern.

### Beispiel 4: Ringöffnung mit AlCl₃ und Eisessig

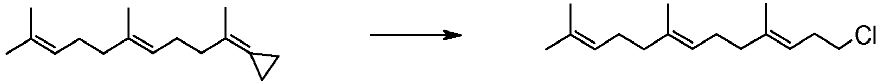

**Einsatzstoffe:**

| **Substanz** | **Reinheit %** | **Molgew. g/mol** | **Masse g** | **Stoffmenge mol** | **Eq** |
|---|---|---|---|---|---|
| C₁₆-Cyclopropan | 94,6 (bez. auf E- & Z) 88,7 (bez. auf E) | 218,38 | 34,5 | 0,15 | 1 |
| Eisessig | 100 | 60,05 | 192,4 | 3,2 | 21 |
| Aluminiumtrichlorid | 100 | 133,34 | 7,27 | 0,055 | 0,36 |

Das C₁₆-Cyclopropan wird bei Raumtemperatur in Eisessig vorgelegt. Aluminiumtrichlorid wird zugegeben. Es wird 17,5 h bei Raumtemperatur gerührt (Umsatzkontrolle via Dünnschichtchromatographie und HPLC). Anschließend wird der Reaktionsansatz mit 600ml Wasser versetzt und 2x mit 200ml Cyclohexan extrahiert. Die organischen Phasen werden vereinigt (pH = 3-4) und mit 120g NaOH (5%ig) gewaschen. Die organische Phase wird über Natrium-sulfat getrocknet und am Rotationsverdampfer bei 50°C/10mbar eingeengt. Man erhält 35,4g Wertprodukt als orangene Flüssigkeit, d.h. bei einer Reinheit von 96,4 HPLC-Flächenprozent in einer Ausbeute von 90% bezogen auf die Summe der Isomere.

**HPLC Methode 2:**

| **Probe :** | | **A** | **Verhältnis** |
|---|---|---|---|
| **RT** | **Substanz** | **FI%** | **E/Z** |
| 10,65 | E-Geranylaceton | 0,4 | |
| 20,35 | 3Z, 7Z-Homofarnesylchlorid | 1,8 | 1 |
| 20,69 | C₁₆-Cyclopropan Z | | |
| 21,29 | 3Z, 7E- Homofarnesylchlorid | 4,4 | 2,4 |
| 22,27 | C₁₆-Cyclopropan E (ident. mit 3E,7Z-Homofarnesylchlorid) | | |
| 22,27 | 3E, 7Z- Homofarnesylchlorid | 24,1 | 1 |
| 23,79 | 3E, 7E- Homofarnesylchlorid | 66,1 | 2,7 |

| | | | |
|---|---|---|---|
| **Σ 96,4 FI%** | | | |

### Beispiel 5 : Substitution des Homofarnesylchlorids zum Homofarnesol

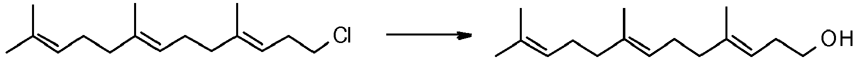

**Einsatzstoffe:**

| **Substanz** | **Reinheit [%]** | **Molgew. [g/mol]** | **Masse / Volumen** | **Stoffmenge [mol]** | **Eq** |
|---|---|---|---|---|---|
| Homofarnesylchlorid | 96,3 (bez. auf alle Homofarnesylchloridisomere) 1 : 2,7 3E,7Z : 3E,7E 1: 2,4 3Z,7Z : 3Z,7E | 254,842 | 12,6 g | 0,0475 | 1 |
| Toluol | | | 10 mL | | |
| Natriumformiat | 97 | 68,01 | 19,4 g | 0,285 | 6 |
| Tetrabutylammoniumbromid | | 322,38 | 2,28 g | 0,0071 | 0,15 |
| NaOH | 25 | 40 | 7,6 g | 0,0475 | 1 |

Homofarnesylchlorid wird bei Raumtemperatur in Toluol vorgelegt. Natriumformiat und Tetrabutylammoniumbromid werden zugegeben. Die Suspension wird auf 110 °C gebracht und 10 h gerührt (Umsatzkontrolle via HPLC; Probenaufarbeitung: 1 ml Reaktionsgemisch wird mit 1 mL NaOH 25%ig 1 h bei Raumtemperatur gerührt. Toluolphase → HPLC). Anschließend wird der Reaktionsansatz mit 25%iger NaOH versetzt und 60 min bei Raumtemperatur gerührt (pH = 10 - 11). Dann wird die vorliegende Suspension auf 300 mL dest. Wasser gegebenen und mit 100 mL Toluol extrahiert. Die organische Phase wird 1 x mit 150 mL, 1 x mit 100 mL VE-Wasser gewaschen, über Natriumsulfat getrocknet und am Rotationsverdampfer bei 50 °C / 10 mbar bis zur Trockene eingeengt. Man erhält 11,1g Wertprodukt als braunes, klares Öl, d.h. bei einer Reinheit von 71,4 HPLC-Flächenprozent in einer Ausbeute von 70% bezogen auf die Summe der Isomere 3E,7E- und 3E,7Z-Homofarnesol.

**HPLC Methode 2:**

| **Probe :** | | | **Verhältnis** |
|---|---|---|---|
| **RT** | **Substanz** | **FI%** | **E/Z** |
| 13,59 | 3E, 7Z-Homofarnesol | 17,6 | 1 |
| 14,79 | 3E, 7E-Homofarnesol | 53,8 | 3,1 |

### Beispiel 6: Chemische Zyklisierung des Homofarnesols zum Ambrox

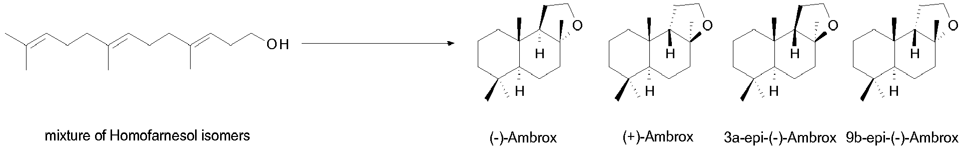

Verschiedene Verfahrensvarianten für eine chemische Cyclisierung sind im Folgenden beschrieben:

### a) Bedingungen: 2-Nitropropan, konz. Schwefelsäure, - 78 °C

**Einsatzstoffe:**

| **Substanz** | **Reinheit %** | **Molgew. g/mol** | **Masse / Volumen** | **Stoffmenge mol** | **Eq** |
|---|---|---|---|---|---|
| Homofarnesol | 90,1 | 236,4 | 1 g | 0,00423 | 1 |
| | (3Z,7E: 5% und 3E,7E: 85,1% chirale HPLC) | | | | |
| Konz. Schwefelsäure | | 98,08 | 3,9 g / 2,12 mL | 0,04 | 9,4 |
| 2-Nitropropan | | 89,09 | 99,2 g / 100 mL | | |

Konz. Schwefelsäure wurde in 50 mL 2-Nitropropan unter Stickstoffatmosphäre bei - 78 °C vorgelegt. Eine Lösung aus 1 g Homofarnesol in 50 mL 2-Nitropropan wurde bei - 78 °C innerhalb von 30 min tropfenweise zugegeben. Die Reaktionskontrolle erfolgte via DC: nach 2 h war das Edukt verbraucht. Zur Aufarbeitung wurde der Reaktionsansatz auf 0 °C gebracht und anschließend langsam in 200 mL gesättigte NaHCO₃-Lösung gegeben. Es wurde dreimal mit 100 mL Diethylether extrahiert. Die organischen Phasen wurden vereinigt und mit 100 mL gesättigter Natriumchloridlösung gewaschen, über Natriumsulfat getrocknet und am Rotationsverdampfer zur Trockene eingeengt. Eine Isomerenmischung von mind. (-) und (+)-Ambrox und 9b-epi-(-)-Ambrox und 3a-epi-(-)-Ambrox (s. oben) wurde erhalten.

### b) Bedingungen: 2-Nitropropan, Trifluormethansulfonsäure, - 78 °C

**Einsatzstoffe:**

| **Substanz** | **Reinheit %** | **Molgew. g/mol** | **Masse / Volumen** | **Stoffmenge mol** | **Eq** |
|---|---|---|---|---|---|
| Homofarnesol | 90,1 | 236,4 | 1 g | 0,00423 | 1 |
| | (3Z,7E: 5% und 3E,7E: 85,1% chirale HPLC) | | | | |
| Trifluormethansulfonsäure | | 150,08 | 6 g / 3,54 mL | 0,04 | 9,4 |
| 2-Nitropropan | | 89,09 | 99,2 g / 100 mL | | |

Trifluormethansulfonsäure wurde in 50 mL 2-Nitropropan unter Stickstoffatmosphäre bei - 78 °C vorgelegt. Eine Lösung aus 1 g Homofarnesol in 50 mL 2-Nitropropan wurde bei - 78 °C innerhalb von 30 min tropfenweise zugegeben. Die Reaktionskontrolle erfolgte via DC: nach 2 h war das Edukt verbraucht. Zur Aufarbeitung wurde der Reaktionsansatz auf 0 °C gebracht und anschließend langsam in 200 mL gesättigte NaHCO₃-Lösung gegeben. Es wurde dreimal mit 100 mL Diethylether extrahiert. Die organischen Phasen wurden vereinigt und mit 100 mL gesättigter Natriumchloridlösung gewaschen, über Natriumsulfat getrocknet und am Rotationsverdampfer zur Trockene eingeengt. Eine Isomerenmischung von mind. (-) und (+)-Ambrox und 9b-epi-(-)-Ambrox und 3a-epi-(-)-Ambrox (s. oben) wurde erhalten.

### c) Bedingungen: 2-Nitropropan, Fluorsulfonsäure, -78 °C

(siehe P. F. Vlad et al. Khimiya Geterotsiklicheskikh Soedinenii, Engl. Transl. 1991, 746)

**Einsatzstoffe:**

| **Substanz** | **Reinheit %** | **Mol-gew. g/mol** | **Masse / Volumen** | **Stoffmenge mol** | **Eq** |
|---|---|---|---|---|---|
| Homofarnesol | 83,3 | 236,4 | 1 g | 0,00423 | 1 |
| | (3Z,7E: 3E,7E = 1 : 15,1) | | | | |
| Fluorsulfonsäure | | 100,07 | 4g | 0,04 | 9,4 |
| 2-Nitropropan | | 89,09 | 99,2 g / 100 mL | | |

Fluorsulfonsäure wurde in 50 mL 2-Nitropropan unter Stickstoffatmosphäre bei - 90 °C vorgelegt. Eine Lösung aus 1 g Homofarnesol in 50 mL 2-Nitropropan wurde bei - 90 °C tropfenweise zugegeben. Es wird weitere 25 h bei - 78 °C gerührt. Zur Aufarbeitung wurde der Reaktionsansatz auf 0 - 5 °C gebracht und anschließend langsam in 200 mL gesättigte NaHCO₃-Lösung gegeben. Es wurde dreimal mit 100 mL Diethylether extrahiert. Die organischen Phasen wurden vereinigt und mit 100 mL gesättigter Natriumchloridlösung gewaschen, über Natriumsulfat getrocknet und am Rotationsverdampfer zur Trockene eingeengt. Eine Isomerenmischung von mind. (-) und (+)-Ambrox und 9b-epi-(-)-Ambrox und 3a-epi-(-)-Ambrox (s. oben) wurde erhalten und via Kugelrohrdestillation getrennt.

Auf die Offenbarung der hierin erwähnten Druckschriften wird ausdrücklich Bezug genommen.

## Patentansprüche

1. Verfahren zur Herstellung von Verbindungen der allgemeinen Formel I 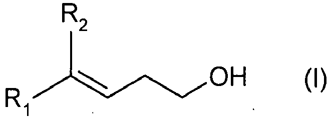 worin
R₁ für einen geradkettigen oder verzweigten, gegebenenfalls ein- oder mehrfach ungesättigten, Kohlenwasserstoffrest mit 1 bis 50 Kohlenstoffatomen steht, wobei die Kohlenwasserstoffkette gegebenenfalls Heteroatome enthält, und R₂ für H oder C₁-C₆-Alkyl steht, wobei man
a) eine Carbonylverbindung der Formel II 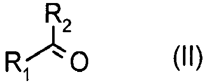 worin R₁ und R₂ die oben angegebenen Bedeutungen besitzen,
mittels Wittig-Olefinierung zu einem Cyclopropan der allgemeinen Formel (III) 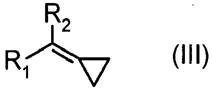 worin R₁ und R₂ die oben angegebenen Bedeutungen besitzen, umsetzt,
b) das Cyclopropan der Formel III unter Ringöffnung zu einer Verbindung der Formel IV 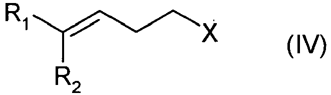 worin R₁ und R₂ die oben angegebenen Bedeutungen besitzen, und X für Halogen oder O-R' steht, worin R' für H, Acyl, Tf-Acetyl oder SO₂-R" steht, worin R" für Alkyl oder Aryl steht;
umsetzt und
c) die Verbindung der allgemeinen Formel IV in die Verbindung der allgemeinen Formel I überführt.

2. Verfahren nach Anspruch 1, wobei man zur Wittig-Olefinierung nach Stufe a) ein Cyclopropylphosphoniumsalz verwendet.

3. Verfahren nach Anspruch 2, worin das Cyclopropylphosphoniumsalz eine Triphenlyphosphonium-Verbindung der Formel V ist 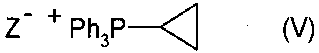 worin Z⁻ für das Anion einer starken Säure, wie insbesondere ein Halogenid, bevorzugt Bromid, steht.

4. Verfahren nach Anspruch 3, wobei man die Verbindung der Formel V herstellt , indem man a) Brombutyrolacton mit Triphenylphosphin umsetzt und anschließend thermisch decarboxyliert oder b) 1,3-Dibrompropan mit Triphenylphosphin umsetzt und anschließend das Umsetzungsprodukt cyclisiert.

5. Verfahren nach einem der vorhergehenden Ansprüche, worin die Ringöffnung der Stufe b) in Gegenwart einer Lewis-Säure bzw. Brönstedtsäure/Protonensäure und eines Nukleophils erfolgt.

6. Verfahren nach Anspruch 5, wobei die Ringöffnung im Wesentlichen stereoselektiv, insbesondere E-selektiv (bezüglich R₁) erfolgt.

7. Verfahren nach einem der vorhergehenden Ansprüche, wobei man in Stufe c) die Verbindung der allgemeinen Formel IV in eine Verbindung der allgemeinen Formel I überführt indem man, wenn X für OR' steht, eine Esterspaltung durchführt, oder wenn X für Halogen steht, das Halogenid in einen Ester, vorzugsweise den Formiatester, überführt und diesen Ester anschließend spaltet.

8. Verfahren nach einem der vorhergehenden Ansprüche, worin man ein Produkt, enthaltend ein (3*E*, 7*E*)-Homofarnesol der Formel la 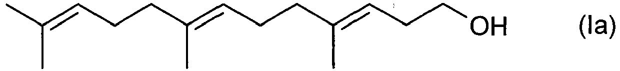 erhält.

9. Verfahren nach Anspruch 8, wobei man in Stufe a) E-Geranylaceton der Formel IIa 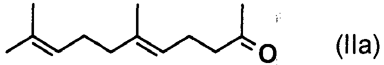 mit Cyclopropylphosphoniumhalogenid umsetzt, so dass man das Cyclopropan der Formel IIIa 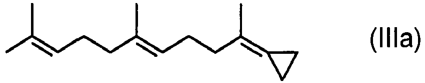 erhält.

10. Verbindung der Formel IIIa.

11. Verfahren zur Herstellung von enantiomerenreinem Ambrox oder einem Stereoisomerengemisch von Ambrox, wobei man (3E, 7E)-Homofarnesol nach einem Verfahren gemäß einem der Ansprüche 1 bis 9 herstellt und das so gebildete Homofarnesol in an sich bekannter Weise chemisch oder enzymatisch zu enantiomerenreinem Ambrox oder einem Stereoisomergemisch von Ambrox umsetzt.

## Claims

1. A process for preparing compounds of the general formula I 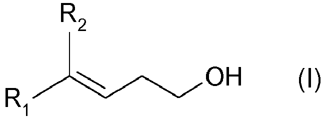 in which
R₁ is a straight-chain or branched, optionally mono- or polyunsaturated hydrocarbon radical having 1 to 50 carbon atoms, where the hydrocarbon chain optionally comprises heteroatoms, and R₂ is H or C₁-C₆-alkyl, where
a) a carbonyl compound of the formula II 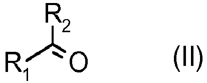 in which R₁ and R₂ have the meanings given above,
is reacted by means of Wittig olefination to give a cyclopropane of the general formula (III) 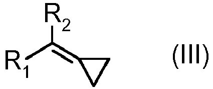 in which R₁ and R₂ have the meanings given above,
b) the cyclopropane of the formula III is reacted, with ring opening, to give a compound of the formula IV 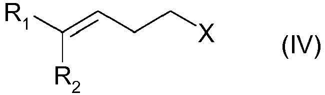 in which R₁ and R₂ have the meanings given above, and X is halogen or O-R', in which R' is H, acyl, Tf-acetyl or SO₂-R", in which R" is alkyl or aryl;
and
c) the compound of the general formula IV is converted to the compound of the general formula I.

2. The process according to claim 1, where a cyclopropylphosphonium salt is used for the Wittig olefination according to stage a).

3. The process according to claim 2, in which the cyclopropylphosphonium salt is a triphenylphosphonium compound of the formula V 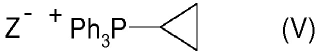 in which Z⁻ is the anion of a strong acid, such as, in particular, a halide, preferably bromide.

4. The process according to claim 3, where the compound of the formula V is prepared by reacting a) bromobutyrolactone with triphenylphosphine and then thermally decarboxylating the reaction product, or b) reacting 1,3-dibromopropane with triphenylphosphine and then cyclizing the reaction product.

5. The process according to any one of the preceding claims, in which the ring-opening in stage b) takes place in the presence of a Lewis acid or Brönstedt acid/protonic acid and a nucleophile.

6. The process according to claim 5, where the ring opening takes place essentially stereoselectively, in particular E-selectively (with respect to R₁).

7. The process according to any one of the preceding claims, where, in stage c), the compound of the general formula IV is converted to a compound of the general formula I by, when X is OR', carrying out an ester cleavage, or when X is halogen, converting the halide to an ester, preferably the formate ester, and then cleaving this ester.

8. The process according to any one of the preceding claims, in which a product comprising a (3E, 7*E*)-homofarnesol of the formula Ia 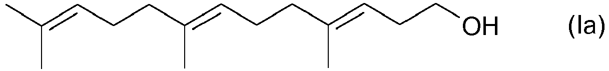 is obtained.

9. The process according to claim 8, where, in stage a), E-geranyl acetone of the formula IIa 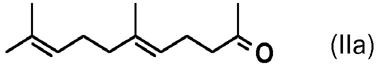 is reacted with cyclopropylphosphonium halogenide, so that the cyclopropane of the formula IIIa 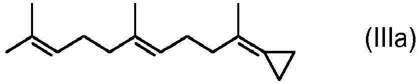 is obtained.

10. A compound of the formula IIIa.

11. A process for preparing enantiomerically pure ambrox or a stereoisomer mixture of ambrox, where (3E, 7E)-homofarnesol is prepared by a process according to any one of claims 1 to 9 and the homofarnesol formed in this way is reacted chemically or enzymatically in a manner known per se to give enantiomerically pure ambrox or a stereoisomer mixture of ambrox.

## Revendications

1. Procédé de fabrication de composés de formule générale I 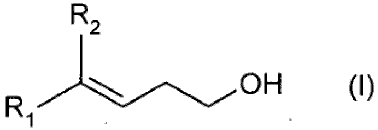 dans laquelle
R₁ représente un radical hydrocarboné linéaire ou ramifié, éventuellement mono- ou polyinsaturé, de 1 à 50 atomes de carbone, la chaîne hydrocarbonée contenant éventuellement des hétéroatomes, et R₂ représente H ou alkyle en C₁-C₆, selon lequel
a) un composé de carbonyle de formule II 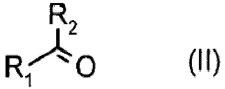 dans laquelle R₁ et R₂ ont les significations indiquées précédemment,
est transformé par oléfination de Wittig en un cyclopropane de formule générale (III) 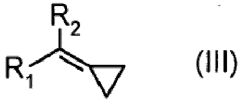 dans laquelle R₁ et R₂ ont les significations indiquées précédemment,
b) le cyclopropane de formule III est transformé par ouverture de cycle en un composé de formule IV 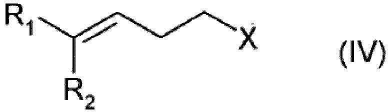 dans laquelle R₁ et R₂ ont les significations indiquées précédemment et X représente halogène ou O-R', R' représentant H, acyle, Tf-acétyle ou SO₂-R'', R'' représentant alkyle ou aryle ;
et
c) le composé de formule générale IV est transformé en le composé de formule générale I.

2. Procédé selon la revendication 1, dans lequel un sel de cyclopropylphosphonium est utilisé pour l'oléfination de Wittig selon l'étape a).

3. Procédé selon la revendication 2, dans lequel le sel de cyclopropylphosphonium est un composé de triphénylphosphonium de formule V 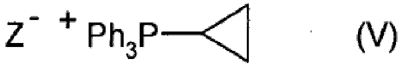 dans laquelle Z⁻ représente l'anion d'un acide fort, tel que notamment un halogénure, de préférence le bromure.

4. Procédé selon la revendication 3, dans lequel le composé de formule V est fabriqué par a) mise en réaction de bromobutyrolactone avec de la triphénylphosphine puis décarboxylation thermique, ou b) mise en réaction de 1,3-dibromopropane avec de la triphénylphosphine, puis cyclisation du produit de réaction.

5. Procédé selon l'une quelconque des revendications précédentes, dans lequel l'ouverture de cycle de l'étape b) a lieu en présence d'un acide de Lewis ou d'un acide de Brönsted/acide protonique et d'un nucléophile.

6. Procédé selon la revendication 5, dans lequel l'ouverture de cycle a lieu de manière essentiellement stéréosélective, notamment E-sélective (par rapport à R₁).

7. Procédé selon l'une quelconque des revendications précédentes, dans lequel, à l'étape c), le composé de formule générale IV est transformé en un composé de formule générale I par, lorsque X représente OR', clivage d'ester ou, lorsque X représente halogène, transformation de l'halogénure en un ester, de préférence l'ester formiate, puis clivage de cet ester.

8. Procédé selon l'une quelconque des revendications précédentes, dans lequel un produit contenant du (3E, 7E)-homofarnésol de formule Ia 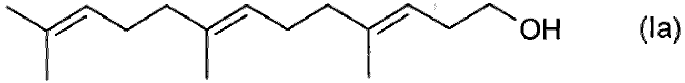 est obtenu.

9. Procédé selon la revendication 8, dans lequel, à l'étape a), de la E-géranylacétone de formule IIa 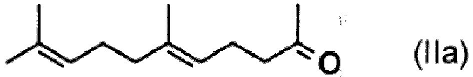 est mise en réaction avec un halogénure de cyclopropylphosphonium, afin d'obtenir le cyclopropane de formule IIIa

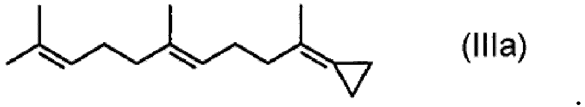

10. Composé de formule IIIa.

11. Procédé de fabrication d'Ambrox énantiomériquement pur ou d'un mélange de stéréoisomères d'Ambrox, selon lequel du (3E, 7E)-homofarnésol est fabriqué par un procédé selon l'une quelconque des revendications 1 à 9, et l'homofarnésol ainsi formé est transformé chimiquement ou enzymatiquement d'une manière connue en soi en Ambrox énantiomériquement pur ou en un mélange de stéréoisomères d'Ambrox.
